# EUROPEAN PATENT APPLICATION

(11) **EP 1 674 849 A2**
(43) Date of publication of application: **28.06.2006**
(21) Application number: 06110462.6
(22) Date of filing: 19.05.1998
(51) Int. Cl.: G01N 1/36, B01J 19/00, G01N 15/14, G06F 17/40

(54) **Method of making high density arrays**

(62) Divisional of application: 01203716.4
(71) Applicant: BioVentures, Inc.,, Murfreesboro Tennessee 37132 (US)
(72) Inventor: HUDSON, Jr., James R., Huntsville,, AL 35801 (US); DAWSON, Elliot P., Murfreesboro,, AL 37130 (US)
(74) Representative: Read, David Graham

(57) **Abstract**

A method of producing high density arrays of target substances comprising the step of sectioning a bundle of target-strands, wherein the target-stands comprise the target substances, and wherein the sectioning in a plurality of high density arrays. Additionally, the method can include additional steps, such as stabilizing the target-strands or bundles, incorporating one or more additional materials into the high density array, and interrogating the high density array. Also, a high density array produced according to the method.

## Description

### BACKGROUND

High density arrays of immobilized natural or synthetic target substances allow the simultaneous screening of analytes for the presence of specific properties. Such high density arrays have proven useful in a variety of technological fields including chemistry, genetics, immunology, material sciences, medicine, molecular biology and pharmacology. For example, high density arrays of nucleic acids are used to ascertain gene sequences, to detect the presence of genetic mutations and to detect the qualitative and quantitative differential expression of gene products. Similarly, high density arrays of peptides are used to map epitopic sequences that elicit immune responses. Further, arrays of target substances are used to identify compounds for the development of pharmaceutical agents.

Currently, methods for the construction of high density arrays of test substances are generally of two types. First, arrays are constructed by individually applying preformed natural or synthetic target substances, such as biomolecules, directly to specific locations on a support. Supports include membranes of nitrocellulose, nylon, polyvinylidine difluoride, glass, silicon or other materials, and the target substances can be immobilized to the support by exposing the support to ultraviolet radiation or by baking the support, among other techniques. One such method is disclosed in Pietu et al., "Novel Gene Transcripts Preferentially Expressed in Human Muscles Revealed by Quantitative Hybridization of a High Density cDNA Array," *Genome Research* (1996) 6: 492-503, incorporated herein by reference in its entirety. Various devices have been devised to automate the application method.

The second method of constructing high density arrays involves synthesizing individual target substances at specific locations in situ on a support. In one version of this method, photosynthetic chemistry is used to simultaneously prepare series of different target substances at unique locations on the support. In another version of this method, target substances are synthesized by physically masking or blocking selected areas on a support and the desired chemical synthesis reaction is carried out on the unmasked portion of the support. Examples of this method are disclosed in, Fodor et al., "Light-Directed, Spatially Addressable Parallel Chemical Syntheses," *Science* (1991) 251:767-777; United States Patent 5,436,327; and Southern, E.M. et al.," Analyzing and Comparing Nucleic Acid Sequences by Hybridization to Arrays of Oligonucleotides; Evaluation using Experimental Models," *Genomics* (1992) 13: 1008-1017, incorporated herein by reference in their entirety.

Both methods of constructing high density arrays are associated with several disadvantages. First, the methods can only produce a relatively limited number of identical arrays at one time. Secondly, it is difficult to check the arrays being produced by these methods during production to determine the integrity of the production steps. Third, many potential target substances cannot be applied to supports and cannot be synthesized in situ on supports by currently used methods. Further, arrays composed of test substances from more than one chemical category, such as arrays of peptides and nucleic acid test substances, are not described. Also, the methods are only capable of producing arrays of target substances in a layer having a relatively limited thickness. Additionally, each method can produce arrays of target substance zone dimensions having only relatively limited sizes.

Therefore, there is a need for an alternate method of producing high density arrays which does not have the disadvantages inherent in the known methods of high density array production. For example, the method should preferably be able to produce large numbers of identical arrays simultaneously, rapidly and cost effectively. The method should be able to use a wide variety of target substances and supports, including test substances and supports that cannot be incorporated into arrays by presently used methods. The method should be able to produce arrays in more than two dimensions, in varying thicknesses and sizes, and in configurations other than a planar configuration. Additionally, the method should be able to produce arrays having a variety of target substance zone dimensions, including dissimilarly sized zones for different target substances in an array. Also, the method should be able to produce high density arrays of test substances from different categories or chemical classes, such as arrays of peptide and nucleic acid test substances. Further, the method should be able to use preformed target substances or to use target substances that are synthesized in situ, as necessary, to incorporate the advantages of these methods.

### SUMMARY

According to one aspect of the present invention, there is provided a method of producing high density arrays of target substances comprising the step of sectioning a bundle of target-strands, wherein the target-strands comprise the target substances, and wherein the sectioning results in a high density array. The method can also comprise a step of stabilizing the bundle, incorporating an additional material into the bundle or interrogating the high density array.

Also, provided is a high density array produced by sectioning a bundle comprising a plurality of target-strands, wherein the plurality target-strands comprise target substances. The high density arrays can have target substances present in one, two or three Cartesian axes.

Further provided is a method of making a high density array comprising the step of rolling a membrane or stacking membranes impregnated with lines target substances to produce a bundle. Also, provided is a method of ascertaining gene sequences, detecting the presence of genetic mutations, detecting the qualitative or quantitative differential expression of gene products, mapping epitopic sequences that elicit immune responses, or identifying compounds for the development of pharmaceutical agents, using the high density arrays produced according to the present invention.

### FIGURES

The features, aspects and advantages of the present invention will become better understood with regard to the followiaig description, appended claims and accompanying figures where:
Figures 1 through 3 depict the production of high density arrays using a bundle of fibers which comprise target substances according to the present invention;
Figures 4 through 5 depict the production of high density arrays using a bundle comprising a membrane having lines of target substances applied on the membrane according to the present invention;
Figures 6 through 8 depict the production of high density arrays using a bundle comprising a plurality of membranes having lines of known target substances applied on the membrane according to the present invention;
Figures 9 through 11 depict the production of high density arrays using a bundle comprising a rolled membrane having lines of known target substances applied on the membrane according to the present invention;
Figures 12 through 14 depict the production of high density arrays using a bundle comprising tubes filled with target substances according to the present invention;
Figure 15 is a photograph of an autoradiograph showing the result of a hybridization study performed on an array produced according to the present invention; and
Figure 16 is a photograph of an autoradiograph showing the result of a hybridization study performed on another array produced according the present invention.

### DESCRIPTION

According to one embodiment of the present invention, there is provided a method of making a high density array of target substances for determining the identity or properties of analytes or for determining the identity or properties of the target substances. According to another embodiment of the present invention, there is provided a high density array of target substances for determining the identity or properties of analytes or for determining the identity or properties of the target substances.

As used herein, the term "target substance" refers to the component of the high density array that potentially interacts with one or more analytes of interest. Target substances can be atoms, molecules, complex chemicals, organelles, viruses, cells or materials, or can be combinations of these entities, or can be other entities as will be understood by those with skill in the art with reference to the disclosure herein. For example, the target substances of a high density array according to the present invention can be selected from one or more of the group of atoms such as zinc, sulfur, and gold; biomolecules such as polynucleotides, DNA, RNA, peptides, proteins, glycoproteins, lipoproteins, carbohydrates, lipids, immunoglobulins, and their synthetic analogs and variants; viruses; sub-cellular components such as microdisected chromosomes and mitochondria; cells including prokaryotic cells, archaebacteria, and eukaryotic cells; and materials such as metallic alloys, ceramics, glasses, semiconductors, superconductors, plastics, polymeric materials, wood, fabric and concrete.

As used herein, the term "analyte" refers to an entity whose identity or properties are to be determined by interaction with the target substances on a high density array according to the present invention. Alternately or simultaneously, the analyte can be used to determine the identity or properties of the target substances by interaction with the target substances on a high density array according to the present invention. Analytes can be selected from the same group as target substances, such as proteins or nucleic acids, or can be a physical or environmental condition such as one or more condition selected from the group consisting of temperature, pH, or salt concentration.

As used herein, the term "target-strand" refers to a strip of target substance. These strips can consist entirely of one or more target substances, or can comprise one or more target substances with a support or a container. The target substances can be absorbed to, adsorbed to, attached to, embedded in, or coated on the support, or contained within the container. For example, the target-strands can include cast rods of target substances such as metal alloys, concrete or plastic, or can include target substances absorbed onto glass fibers or silk threads, attached to polymer fibers, embedded in a porous rod, coated on a metal wire, or contained within a matrix of gelatin. Further, target-strands can include lines of target substances which are written, drawn, printed or embossed on a glass slide or on a membrane such as a thin planar sheet of polymeric substance, or on an equivalent support. Additionally, target-strands can include target substances attached to the inside of tubes.

As used herein, the term "matrix" refers to a material in which target substances can be embedded or to which target substances can be attached to supply additional structural support, to serve as a spacer, to display the target substance to the analyte, or to influence the interaction between the target substance and the analyte such as by electrically insulating target substances from each other. Matrices can be polymeric materials such as one or more substances selected from the group consisting of aerogel, agarose, albumin, gelatin, hydro-gel and polyacrylamide.

As used herein, the term "bundle" refers to an ordered artangement or assembly of target-strands. For example, a bundle can include a stack of target-strands where each target-strand comprises a tube filled with a target substance, or where each target-strand comprises lines of target substances drawn on a membrane, or where each target-strand comprises a wire of a target substance.

### Method of Producing High Density Arrays

The method of producing high density arrays according to the present invention comprises the steps of (a) assembling a bundle of target-strands, and (b) sectioning the bundle to produce an array. Additionally, the method can include a step of stabrizing the target-strands or bundles. Further, the method can include a step of incorporating one or more additional materials into the high density arrays. Also, the method can include a step of interrogating the high density array.

### Assembling a Bundle of Target-Strands

Arraysbundle of target-strands can be produced by a number of methods. For example, a bundle of targels-strand can be produced by first filling tubes with target substances or with target substances in combination with a matrix. The target substance can be enclosed within the matrix without being chemically bound to the matrix or can be attached to the matrix by covalent forces, by ionic forces, by hydrogen bonding or by other forms of attachment. The tubes are then arranged and secured substantially parallel to to their long axes to produce the bundle of target-strands.

A bundle of target-strands can also be produced by first coating or impregnating a support, such as a membrane, fiber, tube, or rod, with a target substance, or by applying solutions of target substance onto a support with a fountain pen nib such as an artist's crow's- quill pen nib or an air-brush, or by ink-jet printing, embossing or thermally transferring solutions of target substances onto a support. Next, these supports are stacked, rolled or folded to produce the bundle of target-strands. The resultant bundle contains rows of target substances that are aligned relatively parallel to the long axis of target substance application.

### Sectioning the Bundles to Produce the Arrays

After assembling, the bundles are sectioned to produce the armys. The bundles can be sectioned with a microtome, laser, saw, hot wire or other cutting device or method as will be understood by those with skill in the art with reference to the disclosure herein. The sectioning can result in a high density array with target substances having any of a wide variety of thicknesses. For example, the array can have target substances with a thickness of between about 0.1 µm to about 1 mm or thicker. Further, unlike prior known methods of producing arrays, the method disclosed herein can readily produce arrays having target substances with a thickness of greater than 50 µm. This is advantageous as it can increase the signal generated by the target substance as compared to signals generated by target substances on thinner arrays.

In a preferred embodiment, the assembled bundle has target-strands which have long axes substantially parallel to each other and the bundle is sectioned substantially perpendicular to the long axes of the target-strands to produce the high density arrays. The sectioning can also be performed at an angle other than substantially perpendicular to the long axes of the target-strands, such as to produce oval arrays from a cylindrical bundle.

Depending on the form of the bundle and the direction of sectioning, the sectioning step can produce high density arrays with one, two or three analytical axes, that is, high density arrays having target substances in one, two or three Cartesian axes. For example, arrays with one analytical axis can result from cross-sectioning a bundle having target substances lying in a single plane. Arrays with two analytical axes can result from cross-sectioning a bundle having target substances lying in a plurality of planes. Arrays with two analytical axes can also be produced by combining multiple, single analytical axis arrays. Arrays with three analytical axes can be produced by combining multiple, single analytical axis arrays, by combining a single analytical axis array with an array with two analytical axes, or by combining a plurality of arrays with two analytical axes.

For example, a high density array with one analytical axis can be produced by sectioning a bundle formed from target-strands made by depositing target substances in parallel lines on a flat membrane, where sectioning is performed in a plane perpendicular to the plane formed by the lines. Similarly, a high density array with two analytical axes can be produced by sectioning a bundle formed from target-strands comprising a stack of membranes, where each membrane has target substances sited in parallel lines, and where sectioning is performed in a plane perpendicular to the long axes of the target substance lines. Further, a high density arrary with three analytical axis can be produced by stacking a plurality of high density array with two analytical axes produced by this sectioning.

### Stabilizing the Bundle of Target-Strands

The method of producircg high density arrays according to the present invention can also include a step of stabilizing the bundle of target-strands. Stabilization can improve the form or the function of the bundle or array, such as making the bundle easier to section, or isolating target substances from each other in the array. The stabilizing step can be performed at any time during or after the assembly of the bundle of target-strands, as is appropriate to the type of stabilization. For example, stabilization can be accomplished by embedding the bundle of target-strands in a matrix, such as epoxy, polypropylene or polystyrene.

### Incorporating Additional Materials into the High Density Arrays

The method of producing high density arrays according to the present invention can also include a step of incorporating one or more additional materials into high density arrays during or after assembly of the bundle of target-strands, including after the sectioning step. These materials can improve the form or the function of the high density array. For example, the incorporation step can include adding antioxidants or microbial inhibitors or other substances to maintain the integrity of the high density array over time.

Further, the incorporation step can include adding substances to the matrix which reduce background noise, such as a nonfluorescent counterstain, or which increase the detection signal. Similarly, the incorporation step can include adding a scintillant to the matrix to facilitate the detection of radioactive analytes. Also, the incorporation step can include adding cofactors necessary for certain modes of detection to the matrix, such as secondary enzymes which are necessary for enzymatic color development, or an energy transfer dye which can enhance the detection of a fluorescent label. Additionally, a surface of a high density array produced by the method disclosed herein can be coated with silver or another reflective material to enhance the amount of light available for detection.

### Interrogating the High Density Arrays

The method of producing high density arrays according to the present invention can also include a step of interrogating the high density arrays. In a preferred embodiment, the interrogating step is selected from the group of visual inspection with or without magnification, chemical deposition, electrical probing, mechanical sensing and magnetic sensing. In another embodiment, the step of interrogating comprises plaaixig the array in close proximity to a collection of interdigitated electrodes and measuring capacitance changes resulting from interactions between the target substances on the high density array and the interdigitated electrodes.

### Production of High Density Arrays from Bundles Comprising Fibers

In one embodiment, high density arrays are produced from bundles of target-starands comprising fibers or threads. The fibers or threads can comprise natural or synthetic material selected from the group consisting of cotton, silk, nylon, and polyester, or can be other materials as will be understood by those with skill in the art with reference to the disclosure herein.

In a preferred embodiment, the bundles of target-strands are produced by directly impregnating fibers with an aqueous solution of the target substance. A series of such fibers are impregnated with different target substances and the identity of each the target substance each fiber contains is recorded in a database. The fibers are washed to elute unbound target substances and are treated with a non-interfering substance to block nonspecific binding sites on the fibers and the immobilized target substances. The fibers are then dried to fix the blocking agent to the fiber and to the immobilized target substances.

The fiber are then assembled into bundles with the location of each fiber and its associated immobilized target substance noted in the database. The bundle of fibers is preferably stabilized by embedding or otherwise impregnating the bundle in a matrix to provide structural support to the bundle.

The bundle is then sectioned substantially perpendicular to the long axis of the fibers using suitable instrumentation to provide a plurality of high density arrays. Preferably, the sectioning results in a plurality of identical high density arrays. The identity and location of the target substances on each array are tracked through the information in the database. These arrays can be utilized to simultaneously screen analytes for the presence of specifc properties, or can be utilized for other purposes as will be understood by those with skill in the art with reference to the disclosure herein.

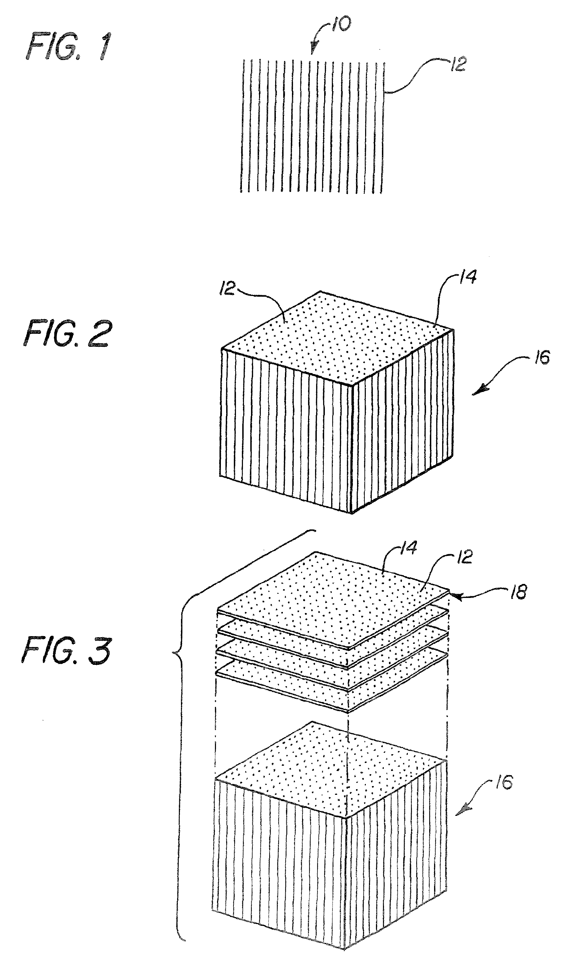

Referring now to Figures 1 to 3, there are shown respectively, target-strands 10 comprising a series of coated fibers 12 impregnated with known target substances; the target-strands 10 embedded in a matrix 14 and assembled into a bundle 16; and the bundle 16 being sectioned to produce a plurality of identical high density arrays 18, where each array has target substances in two analytical axes.

### Production of High Density Arrays from Bundles Comprising Membranes

In one embodiment, high density arrays are produced from bundles comprising membranes. The anesniamnes can comprise thin planar sheets of a polymeric substance, or can comprise other materials as will be understood by those with skill in the art with reference to the disclosure herein.

In a preferred embodiment, the bundles are produced by applying lines of a composition containing the target substances on the membranes by writing, drawing, printing or embossing. The identity and location of each target substance is recorded in a database. The membranes are then treated, if necessary, to fix the target substances to the membrane.

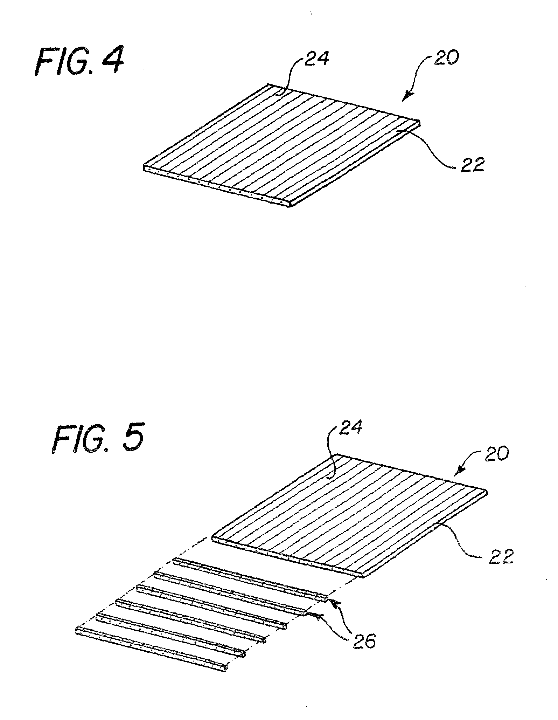

One membrane produced in this manner can be sectioned to produce a plurality of high density arrays, each array having target substances arranged in one analytical axis. Referring now to Figures 4 and 5, there are shown respectively, bundle 20 comprising a membrane 22 having lines of known target substances 24 applied on the membrane 22; and the bundle 20 being sectioned to produce a plurality of high density arrays 26, where each array has target substances arranged in one analytical axis.

Alternately, a plurality of membranes produced in this manner can be assembled into bundles with the identity and location of each immobmmd target substance noted in the database. Assembly can comprise rolling or folding the membrane, or can comprise stacking a plurality of target substance impregnated membranes. If necessary, the bundle is stabilized such as by embedding or otherwise impregnating the bundle in a matrix to provide structural support to the bundle.

The bundle is then sectioned substantially perpendicular to the long axis of the target substance lines on the membranes using suitable instrumentation to provide a plurality of high density arrays, where each array has target substances arranged in two analytical axes. Preferably, the sectioning results in a plurality of identical high density arrays. The location and identity of the target substances are tracked through the information in the database. These arrays can be utilized to simultaneously screen analytes for the presence of specific properties, or can be utilized for other purposes as will be understood by those with skill in the art with reference to the disclosure herein.

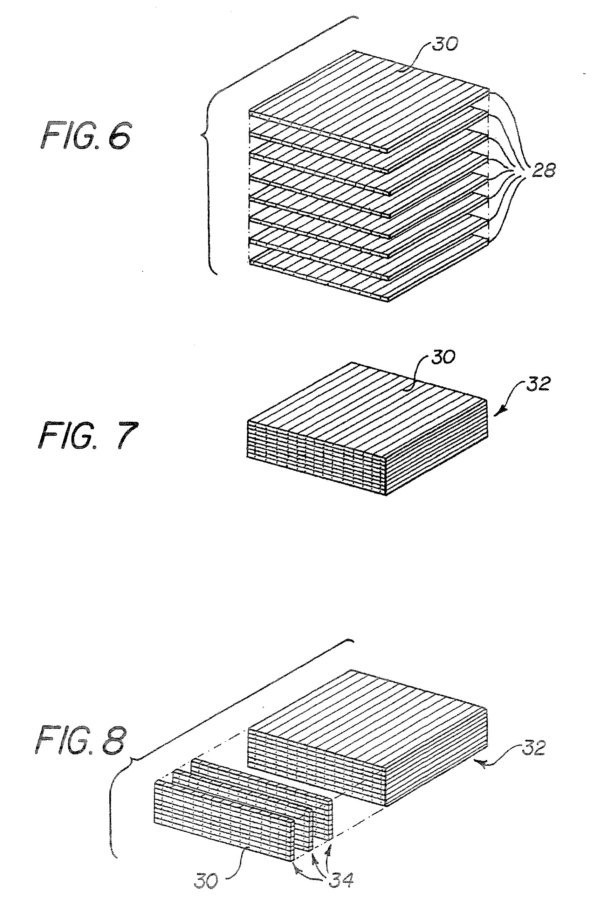

Referring now to Figures 6 to 8, there are shown resgactively, a plurality of membranes 28 having lines of target substances 30 applied on each membranes 28; the membranes 28 stacked and stabilized to form the bundle 32; and the bundle 32 being sectioned to produce a plurality of high density arrays 34, where each array has target substances 28 arranged in two analytical axes.

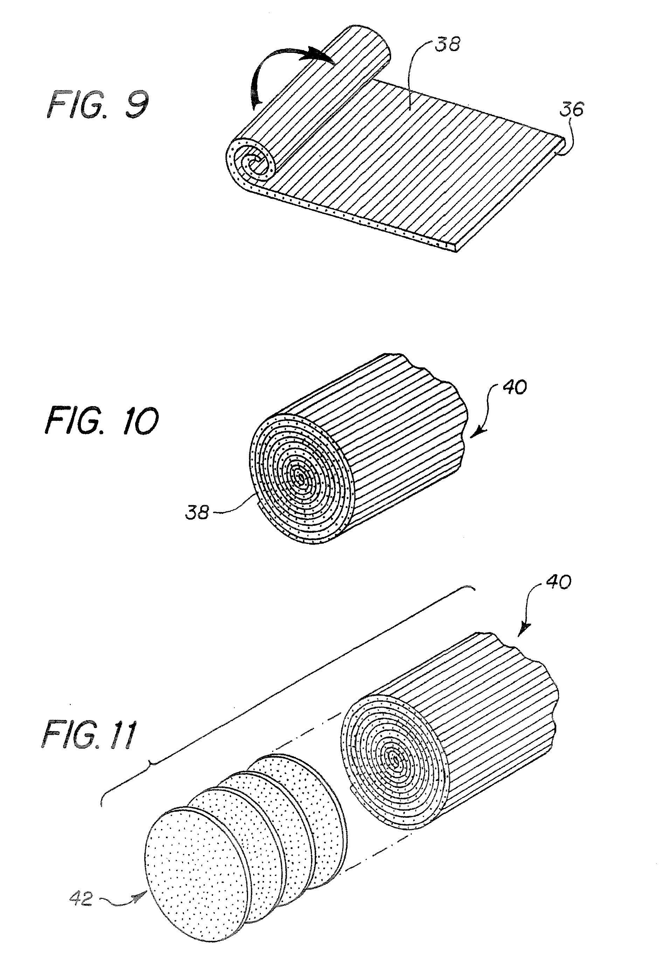

Referring now to Figures 9 to 11, there are shown respectively, a membrane 36 having lines of known target substances 38 applied on membrane 36; the membrane 36 being rolled and stabilized to form a bundle 40; and the bundle 40 being sectioned to produce a plurality of high density arrays 42, where each array has target substances 38 arranged in two analytical axis.

### Production of High Density Arrays from Bundles Comprising Tubes

In one embodiment, high density arrays are produced from target-strands comprising tubes. The tubes can comprise polyimide, nylon, polypropylene, polyurethane, silicone, ethyl vinyl acetate, stainless steel, copper, glass, or fused silica, or can be other materials as will be understood by those with skill in the art with reference to the disclosure herein.

In a preferred embodiment, target-strands are produced by coating the inside of the tubes with an aqueous solution of the target substance such that the target substance is absorbed, adsorbed or covalently bound to the interior surface of the tubes. Alternately, the tubes can be filled with the target substances with or without embedding the target substances in a matrix. A series of such tubes are produced by coating or filling the tubes with different target substances and the identity of each target-strand and the target substance it contains is recorded in a database.

The tubes are then assembled into bundles with the location of each tube and its associated target substance noted in the database. The bundle of tubes is preferably stabilized by embedding the bundle in a matrix to provide structural support to the bundle.

The bundle is then sectioned substantially perpendicular to the long axis of the tubes using suitable instrumentation to provide a plurality of high density arrays. Preferably, the sectioning results in a plurality of identical high density arrays. The identity and location of the target substances are tracked through the information in the database. These arrays can be utilized to simultaneously screen analytes for the presence of specific properties, or can be utilized for other purposes as will be understood by those with skill in the art with reference to the disclosure herein.

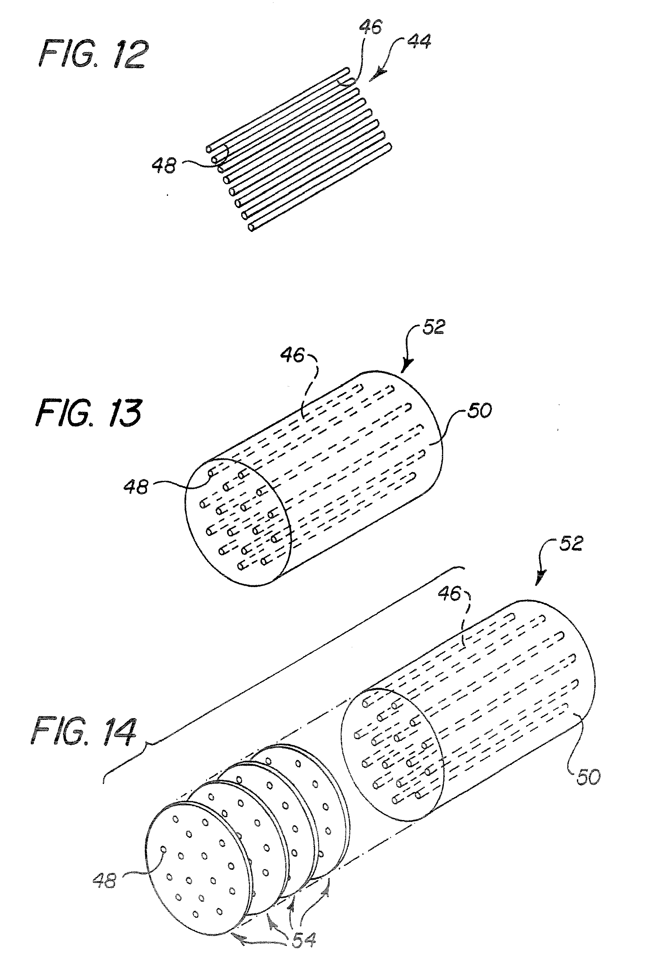

Referring now to Figures 12 to 14, there are shown respectively, target-strands 44 comprising a series of tubes 46 filled with known target substances 48; the target-strands 44 embedded in a matrix 50 and assembled into a bundle 52; and the bundle 52 being sectioned to produce high density arrays 54, where each array has target substances 48 arranged in two analytical axis.

### EXAMPLE I

### Production and Use of High Density Arrays Comprising DNA Coated Threads

The method of producing high density arrays from a bundle comprising fibers or threads according to the present invention is used to produce high density arrays of DNA target substances as follows. Cotton thread is evaluated for wetability by an aqueous solution by dipping the thread in water. Water beading on the surface of the thread indicates that the thread could have binders, oils or other materials on its surface that can negatively affect the wetability of the thread for producing target-strands. If beading occurs during the wetability test, the threads should be washed in methanol, ethanol or another suitable solvent miscible with water to remove the undesirable materials. The threads are then placed in water and the water exchanged several times until each thread is fully wetted.

Next, the threads are transferred into an aqueous solution of a polymeric cadonic substance such as poly L-lysine and allowed to equilibrate with the poly L-lysine solution for a few hours. The threads are removed from the poly L-lysine solution and dried to fix the poly L-lysine to the surface of the threads. After fixation, the threads are washed in buffered solution and the buffer is exchanged several times. The threads are removed from the buffer and allowed to dry.

The threads are then cut into lengths, varying from a centimeter to a few meters, as appropriate to the dimensions of the bundle being constructed. Each thread destined for the bundle is preferably cut to the same length.

Next, each cut thread is placed in contact with a solution of DNA having a specific known sequence that is to be the immobilized target substance. The DNA sequence is preferably different for each thread. The DNA used should preferably be single stranded if it is to be utilized for nucleic acid hybridization studies, but can otherwise be left in double stranded form. The DNA can be from natural sources such as plasmid preparations, yeast artificial chromosomes, BAC libraries, YAC libraries or other DNA libraries such as expressed sequence tags, or can be synthetically produced by the polymerase chain reaction or other synthetic processes. The thread and the DNA solution are incubated for a period ranging from a few minutes to a few hours, as is needed to fully saturate the available binding sites on the thread with DNA.

The DNA coated threads are then dried in an oven at approximately 60°C for a period sufficient to affix the DNA to the threads. Alternatively, the DNA can be fixed to the threads by wetting the dried DNA coated thread with 100% ethanol or methanol for a few minutes and allowing the threads to dry. The identity of each thread and its sequence of immobilized DNA target substance is recorded in a database. Next, the threads are individually washed in a buffer such as 1x TE (10 mM tris, I mM EDTA, pH 7.6) to remove unbound DNA from the thread. The DNA coated threads are again dried.

A bundle of DNA coated threads is then assembled by placing the threads parallel and adjacent to one another with the location of each thread in the bundle and its associated DNA recorded in the database. The bundle of threads is stabilized by embedding it in a matrix such as polymethacrylate, epoxy resins, polyethylene glycol, paraffin waxes, gums, poly acrylamide and other similar materials which can, preferably, be handled in liquid form at elevated temperature or in unpolymerized form suitable for embedding the threads. The embedded threads are allowed to harden or to crosslink to impart a rigid structure to the bundle.

In a preferred embodiment, the threads are prevented from becoming fully impregnated with embedding matrix and sequestering the immobilized DNA by coating the threads with a substance such as gelatin, sucrose or polyvinyl alcohol, to which the matrix is impermeant. This is accomplished by wetting the threads bearing the fixed, immobilized DNA in a solution containing from about 0.01% to about 10% by weight of the substance and allowing the threads to dry before being embedded in the matrix.

The stabilized bundle is then sectioned perpendicular to the long axis of the threads using a microtome or similar device to create a plurality of high density arrays preferably having a thickness of between about 0.1 and 100 microns. Each resultant high density array has the same pattern of DNA sequences in specific spatial regions or zones of the array with the target substances arranged in two analytical axis.

One use for these DNA arrays is to detect labeled DNA sequences in an sample which are complimentary to single stranded DNA target substances in the array by incubating the sample and array under hybridizing conditions for a sufficient period of time for hybridization to occur. Unhybridized DNA is removed by washing. The labels are then detected and the zones providing signal are determined. These zones are compared to the database confining the identity of the DNA target substances on the array to establish the identity of the labeled DNA in the sample.

### EXAMPLE II

### Production and Use of High Density Arrays Comprising Peptide Coated Threads

The method of producing high density arrays from a bundle comprising fibers or threads according to the present invention is used to produce high density arrays of peptide target substances as follows. Cotton thread is evaluated for wetability by an aqueous solution by dipping the thread in water. Water beading on the surface of the thread indicates that the thread could have binders, oils or other materials on its surface that can negatively affect the wetability of the thread for producing target-strands. If beading occurs during the wetability test, the threads should be washed in methanol, ethanol or another suitable solvent miscible with water to remove the undesirable materials. The threads are then placed in water and the water exchanged several times until each thread is fully wetted.

Next, the threads are transferred into an aqueous solution of a polymeric cationic substance such as poly L-lysine and allowed to equilibrate with the poly L-lysine solution for a few hours. The threads are removed from the poly L-lysine solution and dried to fix the poly L-lysine to the surface of the threads. After fixation, the threads are washed in buffered solution and the buffer is exchanged several times. The threads are removed from the buffer and allowed to dry.

The threads are then cut into lengths, varying from a centimeter to a few meters, as appropriate to the dimensions of the bundle being constructed. Each thread destined for the bundle is preferably cut to the same length. Cotton thread is evaluated for wetability by an aqueous solution, transferred into an aqueous solution of a polymeric cationic substance such as poly L-lysine, and allowed to equilibrate with the poly L-lysine solution for a few hours. The threads are removed from the poly L-lysine solution and dried to fix the poly L-lysine to the surface of the threads. After fixation, the threads are washed in buffered solution and the buffer is exchanged several times. The threads are removed from the buffer and allowed to dry.

Next, each cut thread is placed in contact with a dimethylsulfoxide (DMSO) solution of peptide having a specific known sequence which is to be the immobilized target substance. The peptide sequence is preferably different for each thread. Individual peptides for use as target substances are obtained commercially or are made by Merifield synthesis, (such as discussed in Bodanszky, M. and Troust, B. Eds. Principles of Peptide Synthesis, 2nd ed., Springer-Verlag, New York, 1993, incorporated by reference in its entirety), as will be understood by those with skill in the art with reference to the disclosure herein. Each thread and peptide solution are incubated for a period ranging from a few minutes to a few hours, as is needed to fully saturate the available binding sites on the thread with peptide.

The peptide coated threads are blotted free of excess DMSO solution and then incubated with mixed pentanes or an equivalent substance to precipitate the peptides onto the surface of the threads. The peptide coated threads are dried at room temperature or between about 60°C and 70°C, with or without a vacuum. The identity of each thread and its sequence of immobilized peptide target substance is recorded in a database. The peptide coated threads are then washed in aqueous buffer such as 0.01 to 1.0 M tris pH 7.0 or phosphate buffered saline pH 7.0, such as 120 mM sodium chloride, 2.7 mM potassium chloride and 10 mM phosphate (available from Sigma Chemical Co., St. Louis, MO, USA) to remove unbound peptides from the threads and dried again at room temperature or between about 60°C and 70°C, with or without a vacuum.

A bundle of peptide coated threads is then assembled by placing the threads parallel and adjacent to one another with the location of each thread in the bundle and its associated peptide recorded in the database. The bundle of threads is stabilized by embedding it in a matrix such as polymeducrylate, epoxy resins, polyethylene glycol, paraffin waxes, gums, poly acrybmide and other similar materials which can, preferably, be handled in liquid form at elevated temperatsare or in unpolymerized form suitable for embedding the threads. The embedded threads are allowed to harden or to crosslink to impart a rigid structure to the bundle.

In a preferred embodiment, the threads are prevented from becoming fully impregnated with embedding matrix and sequestering the immobilized peptide by coating the threads with a substance such as gelatin, sucrose or polyvinyl alcohol, to which the matrix is impermeant. This is accomplished by wetting the threads bearing the fixed, immobilized DNA in a solution containing from about 0.01 to about 10% by weight of the substance and allowing the threads to dry before being embedded in the matrix.

The stabilized bundle is then sectioned perpendicular to the long axis of the threads using a microtome or similar device to create a plurality of high density arrays preferably having a thickness of between about 0.1 and 100 microns. Each resultant high density array has the same pattern of peptide sequences in specific spatial regions or zones of the array.

One use for these peptide arrays is to detect the presence of antibody analyte in a sample, where the antibody is capable of binding to at least one peptide target substance on the array. The presence of the antibody analytes is determined by incubating the sample and array under suitable conditions for a sufficient period of time for binding between the antibody analyte to occur. Unbound sample is removed by washing. The bound antibody is then detected using biotinylated secondary antibodies and labeled streptavidin detection such as alkaline phosphatase, fluorescein or gold labeled streptavidin, according to techniques known to those with skill in the art, and the identity of the peptide target substances on the zones displaying binding are established by reference to the database. Binding indicates the presence of antibody having an epitopic domain for the peptide in the zone. This binding can be evidence of exposure to or infection by an organism, if the sample was derived from a patient's serum.

### EXAMPLE III

### Production and Use of High Density Arrays Comprising DNA Impregnated on a Membrane

The method of producing high density arrays of target substances according to the present invention was used to create arrays from DNA impregnated on a membrane as follows. The Saccharomyces Genome Database at Stanford University Palo Alto, California, USA was used as a source for identifying naturally existing genomic sequences. Using this information, 16 oligonucleotides having similar melting temperatures were randomly selected from the yeast genome as target substances. Each sequence was between 28 and 35 nucleotides and was synthesized by standard cyanoethylphosphoramidite chemistry according to the method disclosed in Gait, M.J., Ed., *Oligonucleotide Synthesis: A Practical Approach*, IRL Press, Oxford, 1984. Each target substance sequence had 100 thymidine residues at the 3' end to facilitate binding of the oligonucleotide to the membrane. See, for example, Erlich, Henry A. and Bugawan, Teodorica L., *HLA Class II Gene Polymorphism: DNA Typing, Evolution, and Relationship to Disease Susceptibility in PCR Technology: Principles and Applications for DNA Amplification,* Stockton Press, New York, pp. 193-208, 1989, incorporated herein by reference in its entirety. The 16 target substances labeled #1 through #16, were individually dissolved in diethylpyrocarconate treated water to a final concentration of 10 ug/µl.

The target substances were applied using an application nib having a reservoir with a capacity of 11 µl connected to the tip by a small capillary channel. The nib was used to draw lines of target substances approximately 1 mm to 3 mm apart on 20 cm x 20 cm membranes of Hybond™ N+charged nylon membranes (Amersham, Arlington Heights, IL, USA). The nib reservoir was filled with 10.5µl of a solution of the first of the 16 target substances using an Eppendorf® 2-10 µl pipetor.

The first membrane, membrane #1, was placed on a clean, flat tabletop with the sheet of a waxed paper larger than the membrane that was used as a separator in the manufacture's packaging placed between the membrane and the tabletop. The nib was aligned such that both sides of the capillary channel touched the waxed paper about 1 cm from the edge of the membrane and the nib was smoothly drawn across the waxed paper and membrane manually using a ruler as a guide to draw a straight line of target substance parallel to one edge of the membrane. The solution of target substance was drawn out of the nib and exhausted after drawing a line approximately 12-16 cm long. This cycle was repeated for each solution of target substance on the first membrane until membrane #1 comprised 16 parallel lines of different DNA target substances approximately I mm to 3 mm apart from each other.

This procedure was repeated to produce two additional membranes, membranes #2 and #3, except that each solution of DNA target substance was applied three times consecutively resulting in a total of 48 parallel lines of target substances on membrane #2 and #3. Each line of target substance was labeled for identification purposes on all of the membranes.

The membranes comprising the lines of DNA target substances were allowed to air dry for about 2 hours and were then crosslinked by application of 1200 µjoules of UV electromagnetic radiation for 35 seconds using a Stratagene 2400 Stratalinker® (Stratagene, La Jolla, CA, USA). Starting with the edge of the membrane containing the leading edge of the target substance lines, one strip about 2 cm in width by 20 cm in length was cut from each of the three membranes so that the lines of target substances were parallel to the 2 cm edge of the strips.

Radioactively labeled DNA probes which were complimentary to the sequence of target substances #1 and #7 were prepared using standard techniques. Hybridization was attempted between the radioactively labeled probes and ad array produced from membrane #1 using standard techniques. In summary, the DNA oligonucleotides was labeled using the Ready to Go Kinase™ kit (Pharmacia, Piscataway, NJ, USA) using gamma-³²P-ATP (ICN Radiochemicals, Irvine, CA, USA) according to the manufacturer's instructions. The labeled probes were purified using Nick™ columns (Pharmacia) according to the manufacturer's instructions, and diluted to approximately 1x10⁶ cpm/ml.

Prehybridization and hybridization was performed using 10 ml HyperHyb™ buffer (Research Genetics, Inc. Huntsville, AL, USA) according to the manufacturer's instructions in a Mini-6™ bybridization oven (Hybaid, Ltd., Middlesex, UK) at 42°C for one hour each. Post-hybridization washes were performed using three 10 ml washes for 15 minutes each in lxSSC, 0.01% sodium dodecyl sulfate (SDS) at 42°C. A final wash was performed in 100 ml of the 1xSSC (0.15 M NaCl, 0.015 M sodium citrate, pH 7.2) (Research Genetics), 0.01 %SDS buffer (Sigma) at 42°C for 15 minutes. A final rinse was performed in 10 ml 1xSSC buffer. The membranes were then air dried for 1-2 hours at room temperature.

Autoradiography was performed by placing the arrays in contact with Biomax™ MS or MR x-ray film (Eastman Kodak, Rochester, NY, USA) at room temperature for between about ½ hour to 4 hours until the desired image intensity was obtained. All probes hybridized with the appropriate target substance on the array demonstrating that the DNA target substances were attached to the membrane and available for probing, and that such probing gave specific, non-ambiguous hybridization results.

Next, the remaining 20 cm by 18 cm portion of membranes #1, 2 and 3 were used to produce arrays as follows. The membranes were immersed in 3% teleostean gelatin (Sigma) in deionized water and were incubated overnight at room temperature to block the membranes. The membranes were then washed three times in 600 ml of deionized water to remove unbound gelatin. The membranes were blotted free of excess moisture between two sheets of 903 blotting paper (Schleicher and Schuell, Keene, NH, USA) and allowed to air dry at room temperature overnight.

Next, a 2 cm by 20 cm strip was cut from each of the three membranes #1, #2, #3 perpendicular to the lines of target substances with the 2 cm edge parallel to the lines of target substances. Each of the strips was tightly rolled about an axis parallel to the lines of target substances to produce a cylinder with the portion of the membrane which did not have target substances applied to it being the innermost part of the cylinder. Clear nail polish was used to seal the free 3 mm edge of the strips to prevent the cylinder from unwinding. Each cylinder was immersed into a plastic bulb 1.25 cm by 7.5 cm filled with unpolymerized LR White™ soft embedding media (Sigma) prepared according to the manufacturer's instructions until the cylinder became fully impregnated by the media. Each cylinder was then placed at the base of the media filled bulb, centered and allowed to palymerize overnight at 60°C. Each bulb containing an embedded cylinder was removed and placed at ambient temperature and polymerization was observed to be complete.

A plurality of arrays approximately 10 microns thick was then produced by repeated sectioning each embedded cylinder perpendicular to its long axis, that is perpendicular to the long axis of each line of target substance. The sectioning was accomplished using a hand microtome, model DK-10 (Edmund Scientific, Barrington, NJ, USA).

Radioactively labeled DNA probes which were complimentary to the sequence of target substances #1 and #7 were prepared using standard techniques. Hybridization was attempted between the radioactively labeled probes and an array produced from membrane #1 using standard techniques. In summary, the DNA oligonucleotides were labeled using the Ready to Go Kinase™ kit (Phamiacia, Piscataway, NJ, USA) using gamma-³²P-ATP (ICN Radiochemicals, Irvine, CA, USA) according to the manufacturer's instructions. The labeled probes were purified using Nick™ columns (Pharmacia) according to the manufacturer's instructions, and diluted to 1x10⁶ cpm/ml.

Prehybridization and hybridization was performed using 10 ml HyperHyb™ buffer (Research Genetics, Inc. Huntsville, AL, USA) according to the manufacturer's instructions in 1.5 ml screw-cap microcentrifuge tubes at 42 C for one hour in a Mini-6 hybridization oven (Hybaid, Ltd., Middlesex, UK) at 42°C. Post-hybridization washes were performed using three 1.5 ml washes for 15 minutes each in 1xSSC, 0.01 % sodium dodecyl sulfate (SDS) at 42°C. A final wash was performed in 100 ml of the 1xSSC (0.15 M NaCl, 0.015 M sodium citrate, pH 7.2) (Research Genetics), 0.01 %SDS buffer (Sigma) at 42°C for 15 minutes a final rinse was performed in 10 ml 1xSSC buffer. The arrays were then air dried for approximately 15-30 minutes. Autoradiography was performed by placing the arrays in contact with Biomax™ MS or MR x-ray film (Eastman Kodak, Rochester, NY, USA) at room temperature for between about ½ hour to 4 hours until the desired image intensity was obtained. Photographs of the developed autoradiographies were then made.

Hybridization was attempted between the radioactively labeled probes and an array produced from membrane #1 using standard techniques. All probes hybridized with the appropriate target substance on the array demonstrating that the DNA target substances were attached to the membrane and available for probing, and that such probing gave specific, non- ambiguous hybridization results.

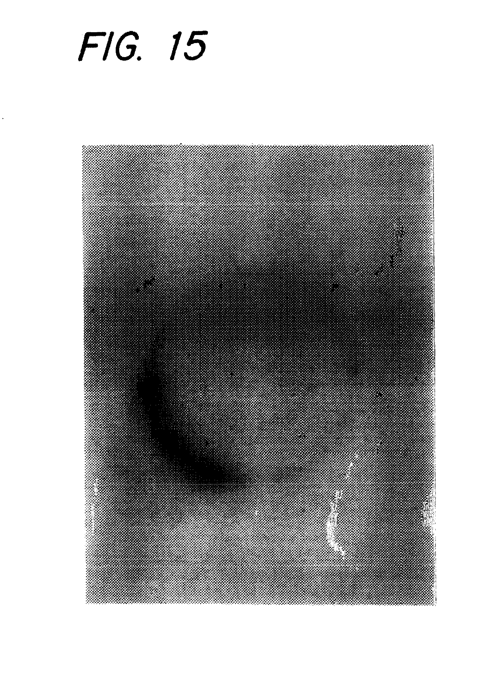

The arrays were tested for functionality as follows. A radioactively labeled DNA probe complimentary to target substance #1 was used to probe an array produced from membrane #2. Referring now to Figure 15, there can be seen a photograph of the autoradiograph of the result. As can be seen, hybridization between the probe and three zones on the array containing target substance #1 occurred, with minimal cross hybridization for the other 45 zones the remaining 15 DNA target substances. Hence, the array demonstrated both functionality for hybridization studies as well as specificity.

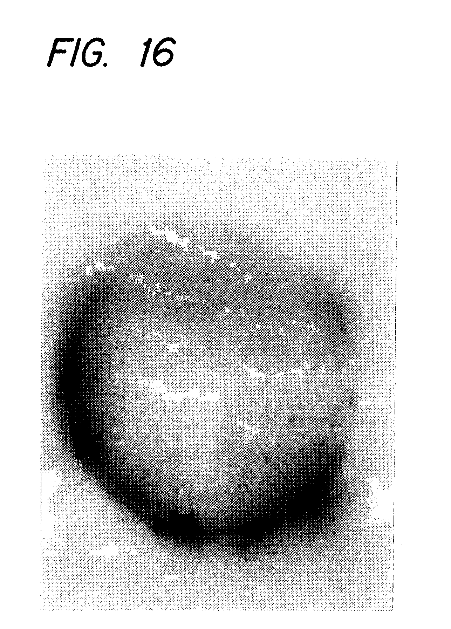

Next, an array produced from membrane #3 was probed with radioactively labeled DNA complimentary to target substances #1 and #7. Referring now to Figure 16, there can be seen an autoradiograph Vt the result. As can be seen, hybridization between the probes and six zones on the array occurred, with minimal cross hybridization for the other 42 zones representing the remaining 14 DNA target substances.

Although the present invention has been discussed in considerable detail with reference to certain preferred embodiments, other embodiments are possible. Therefore, the scope of the appended claims should not be limited to the description of preferred embodiments contained herein.

## Claims

1. A method of producing high density arrays of target substances comprising the steps of sectioning a bundle of target-strands;
wherein the target-strands comprise the target substances;
wherein the location of each target substance within the bundle is noted in a database; and
wherein the sectioning results in a high density array.

2. A method as claimed in claim 1, further including the step of stabilizing the bundle.

3. A method as claimed in claim 1 or 2, further including the step of incorporating an additional material into the bundle.

4. A method as claimed in claim 1, 2 or 3, further including the step of interrogating the high density array.

5. A method as claimed in any one of claims 1 to 4, wherein at least one of the target substances in the sectioning step is selected from the group consisting of zinc, sulfur, gold, polynucleotides, DNA, RNA, peptides, proteins, glycoproteins, lipoproteins, carbohydrates, lipids, immunoglobulins, viruses, chromosomes, mitochondria, prokaryotic cells, archaebacteria, eukaryotic cells, metallic alloys, ceramics, glasses, semiconductors, superconductors, plastics, polymeric materials, wood, fabric and concrete.

6. A method as claimed in any one of claims 1 to 5, wherein the bundle in the sectioning step comprises a target-strands selected from the group consisting of a cast rod of target substance, a target substance absorbed onto a glass fiber, a target substance absorbed onto a silk thread, a target substance attached to a polymer fiber, a target substance embedded in a porous rod, a target substance coated on a metal wire, a target substance contained within a matrix of gelatin, a line of a target substance drawn on a glass slide, a line of a target substance drawn on a membrane, and a target substance attached to the inside of a tube.

7. A method as claimed in any one of the preceding claims, wherein the sectioning is performed with a cutting device selected from the group consisting of a microtome, laser, saw, and hot wire.

8. A method as claimed in any one of the proceeding claims, wherein the sectioning is performed such that the resultant high density array has a thickness of from about 0.1 µm to about 1.0 mm.

9. A method as claimed in any one of claims 1 to 7, wherein the sectioning is performed such that the resultant high density array has a thickness of grater than 50 µm.

10. A method as claimed in claim 2, wherein the stabilizing step is performed by embedding the bundle in a material selected from the group consisting of epoxy, polypropylene and polystyrene.

11. A method as claimed in claim 3, wherein the additional material is selected from the group consisting of an antioxidant, a microbial inhibitor, a nonfluorescent counterstain, a secondary enzyme and a reflecting substance.

12. A method as claimed in claim 4, wherein the interrogating step comprises an activity selected from the group consisting of visual inspection, chemical deposition, electrical probing, mechanical sensing, magnetic sensing and measuring capacitance changes resulting from interactions between the target substances on the high density array and interdigitated electrodes.

13. A high density array produced according to claim 1.

14. A high density as claimed in claim 13, wherein the target substances are present in one Cartesian axis.

15. A high density array as claimed in claim 13, wherein the target substances are present in two Cartesian axes.

16. A high density array as claimed in claim 13, wherein the target substances are present in three Cartesian axes.

17. A high density array of target substances produced by sectioning a bundle comprising a plurality of target-strands, wherein the plurality target-strands comprise target substances, and wherein the location of each target substance within the bundle is noted in a database.

18. A high density array as claimed in claim 17, wherein at least one of the target substances is selected from the group consisting of zinc, sulfur, gold, polynucleotides, DNA, RNA, peptides, proteins, glycoproteins, lipoproteins, carbohydrates, lipids, immunoglobulins, viruses, chromosomes, mitochondria, prokaryotic cells, archaebacteria, eukaryotic cells, metallic alloys, ceramics, glasses, semiconductors, superconductors, plastics, polymeric materials, wood, fabric and concrete.

19. A high density array as claimed in claim 17 or 18, wherein the bundle comprises a target-strand selected from the group consisting of a cast rod of target substance, a target substance absorbed onto a glass fiber, a target substance absorbed onto a silk thread, a target substance attached to a polymer fiber, a target substance embedded in a porous rod, a target substance coated on a metal wire, a target substance contained within a matrix of gelatin, a line of a target substance drawn on a glass slide, a line of a target substance drawn on a membrane, and a target substance attached to the inside of a tube.

20. A high density array as claimed in claim 17, 18 or 19, further comprising a substance selected from the group consisting of epoxy, polypropylene and polystyrene.

21. A high density array as claimed in any one of claims 17 to 20, further comprising a material selected from the group consisting of an antioxidant, a microbial inhibitor, a nonfluorescent counterstain, a secondary enzyme and a reflecting substance.

22. A high density array as claimed in any one of claims 17 to 21, wherein the target substances are present in one Cartesian axis.

23. A high density array as claimed in any one of claims 17 to 21, wherein the target substances are present in two Cartesian axes.

24. A high density array as claimed in any one of claims 17 to 21, wherein the target substances are present in three Cartesian axes.

25. A high density array as claimed in any one of claims 17 to 24, having a thickness of from about 0.1 µm to about 1.0 mm.

26. A high density array as claimed in any one of claims 17 to 24, having a thickness greater than 50 µm.

27. A method of ascertaining gene sequences, detecting the presence of genetic mutations, detecting the quantitative differential expression of gene products, mapping epitopic sequences that elicit immune responses, or identifying compounds for the development of pharmaceutical agents, the method comprising the step of providing a high density array as claimed in any one of claims 17 to 26.
